Europäisches Patentamt

European Patent Office

Office européen des brevets

(11) Publication number: **0 186 371**
**A2**

(12) **EUROPEAN PATENT APPLICATION**

(21) Application number: **85308963.9**

(22) Date of filing: **10.12.85**

(51) Int. Cl.⁴: **G 01 N 33/577**
**G 01 N 33/576**
**//G01N33/535, A61K39/395**

(30) Priority: **11.12.84 GB 8431170**

(43) Date of publication of application:
**02.07.86 Bulletin 86/27**

(84) Designated Contracting States:
**DE FR GB IT SE**

(71) Applicant: **MEDICAL AND SCIENTIFIC RESEARCH ASSOCIATES**
**1 University Plaza Suite 404**
**Hackensack New Jersey 07601(US)**

(72) Inventor: **Wright, Bruce William**
**48 Bush Close**
**Comberton Cambridgeshire(GB)**

(72) Inventor: **Cox, Peter John**
**Church Cottage Church Road Chevington**
**Bury St. Edmunds Suffolk(GB)**

(72) Inventor: **Noyes, Alice Margaret**
**68 High Street**
**Willingham Cambridgeshire(GB)**

(72) Inventor: **Widdows, Danny**
**14 Sherbourne Close**
**Cambridge(GB)**

(72) Inventor: **Saxby, Simon James Yvon**
**1 Bure Close St. Ives**
**Huntingdon Cambridgeshire(GB)**

(74) Representative: **Perry, Robert Edward et al,**
**GILL JENNINGS & EVERY 53-64 Chancery Lane**
**London WC2A 1HN(GB)**

(54) **Monoclonal antibodies specific to antigens of Hepatitis B virus.**

(57) An enzyme-labelled monoclonal antibody, in which the antibody has high binding affinity, is of immunological class IgG, is specific for the Hepatitis B surface antigen "a" determinants, and does not bind to the known biological "a" determinant corresponding to amino-acid sequence 139 to 147 of the Hepatitis B surface antigen.

A labelled antibody of this type can be used in an immunoassay.

EP 0 186 371 A2

Croydon Printing Company Ltd

## BACKGROUND OF THE INVENTION

Of current interest in the fields of analysis and diagnosis is the use of monoclonal antibodies to determine the presence of antigens or viruses in specimens such as urine, blood, water, milk, and the like.

More particularly, monoclonal antibodies specific for the surface antigens of Hepatitis B are desired which when used will rapidly diagnose the presence of such antigens in specimens to detect the presence of the disease.

Hepatitis B virus is transmitted through contact with the serum or blood of a person who has, or has had, the disease. Hepatitis B is further divided into serotypes based on antigens, and includes the Hepatitis B surface antigen(s) (HBsAg), the HBeAg antigen, and Hepatitis B core antigen. In pregnant mothers tests may be required at the time of delivery where the infant is exposed to Hepatitis B. Hepatitis B screening is also important in blood tranfusion and blood products and also in vaccination programs. Hepatitis B surface antigen is currently diagnosed by immunological techniques for the detection of circulating antigen in the blood. A test combining speed, accuracy, and ease of performance will prevail over the present methods which require expensive, slow, labor intensive cultures and other tests. The ability of monoclonal antibodies specifically to bind to antigens of Hepatitis can provide many opportunities for diagnosis and treatment. Such specificity is a most important requirement for proper and accurate analysis and/or diagnosis, particularly in diagnosing the presence of diseases which require prompt treatment.

Hepatitis has been the subject of extensive study to the point where the entire genome of the Hepatitis B virus has been gene sequenced and the corresponding amino acid sequences of the core antigen HBeAg and the surface antigen determined. The surface antigen is of primary importance because its appearance and subsequent disappearance from the serum or plasma of infected individuals is a reliable indicator of the presence of the disease state and its subsequent remission.

It has been shown that the surface antigen consists of a peptide of 226 amino acid residues which have been numbered from 1-226 beginning from the amino terminal end. The immunogenic or hydrophilic antibody binding region is known to span residues 110-160 and is referred to generally as the "a" determinant. In fact, it is presently believed that the group "a" determinant, 110-160, is really composed of at least three overlapping sequences with residues 110-137 specifying the major "d" and "y" subtype system. Residue 139 to 147 specify the biological "a" determinant and at present no subtypes or substitutions in this sequence are believed to occur. The Hepatitis B surface antigen has been categorized into four major subtypes; adw, adr, ayw, and ayr, having the type specific "a" determinant in common. The amino and substitutions responsible for subtype specifically occur in the region from 110-137 and the type specific biological "a" determinant has been localized to a sequence of eight amino acids running from 139-147.

Chemically synthesized peptides corresponding to sequences 139 to 147 and 124 to 137 have been made and these are used to determine where a particular antibody will bind with the antigen.

It has now become clear that the specificity of a monoclonal antibody for a specific epitope or amino acid sequence of the Hepatitis B surface antigen is important particularly because it is known that some epitopes are instrumental

**0186371**

in inducing the formation of antibodies in the affected host, while other epitopes play no role in inducing antibody formation, yet serve as reliable indicators of Hepatitis B infection. This is particularly important in developing vaccines against Hepatitis B infection, as well as detecting and predicting antibody response.

A wide variety of isotopic and nonisotopic immunoassays have been utilized in conjunction with monoclonal antibodies to test for the presence of an antigenic substance. At the present time, agglutination, immuno-fluorescent, chemiluminescent or fluorescent immunoassay, immuno-electron microscopy, radiometric assay systems, radio immunoassays, and enzyme-linked immunoassays are the most common techniques used with the monoclonal antibodies. Other techniques include bioluminescent, fluorescence polarization, and photon-counting immunoassays.

When utilizing the enzyme-linked immunoassay procedure (EIA), it is necessary to bind, or conjugate, the monoclonal antibody with an enzyme capable of functioning in such assay; such as alkaline phosphatase.

The enzyme-linked monoclonal antibody can then be used in the known enzyme-linked immunosor-

**0186371**

bent assay procedure to determine the presence of an antigenic substance.

After the specific antigen is identified, the serotype of the infecting virus can be determined, and appropriate treatment can then be initiated to rapidly and efficiently eliminate the disease.

The production of monoclonal antibodies is now a well-known procedure first described by Kohler and Milstein (Eur. J. Immunol. 6, 292 (1975)). While the general technique of preparing hybridomas and the resultant monoclonal antibodies is understood, it has been found that preparing a specific monoclonal antibody to a specific antigen is difficult, mainly due to the degree of specificity and variations required in producing a particular hybridoma.

## SUMMARY OF THE INVENTION

The present invention provides novel monoclonal antibodies for use in accurately and rapidly diagnosing samples for the presence of Hepatitis B surface antigens.

Briefly stated, the present invention comprises labeled monoclonal antibodies specific for the surface antigen of Hepatitis B virus (HBsAg), consisting essentially of a high binding affinity monoclonal antibody of immunological class IgG specific to HBsAg "a" determinants, said antibody not binding to the known biological "a" determinant corresponding to amino acid sequence 139 to 147 of HBsAg and labeled with an enzyme capable of conjugating with said antibody and of being used in an enzyme-linked immunoassay procedure.

A major advantage of the present invention is the high affinity of the antibodies which yields greater sensitivity and specificity in laboratory diagnosis. This permits lower levels of viral antigens to be accurately detected in clinical specimens; something not possible with presently available Hepatitis monoclonal antibodies. Moreover, the antibodies while binding to HBsAg "a" determinants which serve as an indication of infection with Hepatitis B virus, said monoclonal antibodies not binding to the known biological "a" determinant.

The invention further comprises the process for diagnosing the presence of Hepatitis B surface antigens in a specimen comprising contacting said specimen with the labeled monoclonal antibody in an appropriate immunoassay procedure.

## DETAILED DESCRIPTION

The monoclonal antibodies of the present invention are prepared by fusing spleen cells, from a mammal which has been immunized against the Hepatitis B surface antigen, with an appropriate myeloma cell line, preferably NSO (uncloned), P3NS1-Ag4/1, or Sp2/0 Ag14. The resultant product is then cultured in a standard HAT (hypoxanthine, aminopterin, and thymidine) medium. Screening tests for the specific monoclonal antibodies are employed utilizing immunoassay techniques which will be described below.

The immunized spleen cells may be derived from any mammal, such as primates, humans, rodents (i.e., mice, rats, and rabbits), bovine, ovine, canine, or the like, but the present invention will be described in connection with mice. The mouse is first immunized by injection of the Hepatitis B antigen generally for a period of approximately eleven weeks. When the mouse shows sufficient antibody production against the antigen, as determined by conventional assay, it is given a booster injection of the Hepatitis B antigen, and then killed so that the immunized spleen may be removed. The fusion can then be carried out utilizing immunized spleen cells and an appropriate myeloma cell line.

The fused cells yielding an antibody which give a positive response to the presence of the Hepatitis B surface antigen are removed and cloned utilizing any of the standard methods. The monoclonal antibodies from the clones are then tested against standard antigens referred to as Hepatitis B surface antigens known to contain the "a" determinant. The monoclonal antibody selected, which is specific for the Hepatitis B surface antigen, is then bound to an appropriate enzyme label.

Amounts of antibody sufficient for labeling and subsequent commercial production are produced by the known techniques, such as by batch or continuous tissue culture or culture in vivo in mammals, such as mice.

The present invention will be described with reference to the use of alkaline phosphatase as the enzyme label, it being understood that other suitable enzymes for this purpose include glucose oxidase, galactosidase, peroxidase, urease, and the like.

Such linkage with enzymes can be accomplished by any one of the conventional and known methods, such as the Staphylococcal Protein A method, the glutaraldehyde method, the benzoquinone method, or the periodate method.

Once the labeled monoclonal antibody is formed, testing is carried out employing one of a wide variety of conventional enzyme immunoassay methods. The particular method chosen will vary according to the monoclonal antibody and the label chosen. At the present time, enzyme immunoassays are preferred due to their low cost, reagent stability, safety, sensitivity, and ease of procedure. One example is enzyme-linked immunosorbent assay (EIA). EIA is a solid phase assay system which is similar in design to the radiometric assay, but which utilizes an enzyme in place of a radioactive isotope as the immunoglobulin marker.

Monoclonal diagnostics which detect the presence of Hepatitis B antigen can be used in diagnosing the presence of such antigen in specimens such as urine, blood, stool, water, milk, and the like.

The invention will be further illustrated in connection with the following example which is set forth for purposes of illustration only and not by way of limitation.

## EXAMPLE

A monoclonal antibody was prepared generally according to the method of Kohler and Milstein (Eur. J. Immunol. $\underline{6}$, 292 (1975)).

A.  Antigen Preparation

Surface (HBsAg) antigen of the Hepatitis B virus was used and was prepared by Dr. Colin Howard of the London School of Hygiene and Tropical Medicine.

B.  Animal Immunization

Six Balb/c mice were injected with the prepared antigen. They were given one intraperitoneal injection per week (5 ug/injection) of Hepatitis B virus surface antigen for a total of three weeks followed by two weekly intravenous injections, plus one in Freunds Complete Adjuvant (F.C.A.) after one week. The mice were then be bled approximately six days after the last injection and the serum tested for antibodies by assay. The conventional assay used for this serum titer testing was the enzyme-linked immunosorbent assay system. When the mice showed antibody production after this regimen, generally a positive titer of at least 10,000, a mouse was selected as a fusion donor and given a booster injection (0.02 ml of $10^9$ cells/ml) intravenously three days prior to splenectomy.

C.  Cell Fusion

Spleen cells from the immune mice were then harvested three days after boosting by conventional techniques. First the donor mouse selected was killed and surface sterilized by immersion in 70% ethyl alcohol. The spleen was then removed and immersed in approximately 2.5 ml of Dulbeccos Modified Eagles Medium (DMEM) to which had been added 3% fetal calf serum (FCS). The spleen was then gently homogenized in a LUX homogenizing tube until all cells have been released from the membrane and the cells washed in 5 ml 3% FCS DMEM. The cellular debris was then allowed to settle and the spleen cell suspension placed in a 10 ml centrifuge tube. The debris was then rewashed in 5 ml 3% FCS DMEM. Fifty mls of suspension were then made in 3% FCS DMEM.

The myeloma cell line used was NS0 (uncloned), obtained from the MRC Laboratory of Molecular Biology in Cambridge, England. The myeloma cells were in the log growth phase, and rapidly dividing. Each cell line was washed using a tissue culture medium DMEM containing 3% fetal calf serum.

The spleen cells were then spun down at the same time that a relevant volume of myeloma cells were spun down (room temperature for 7 minutes at 600 g), and each resultant pellet was then separately resuspended in 10 ml 3% FCS DMEM. In order to count the myeloma cells, 0.1 ml of the suspension was diluted to 1 ml and used with a haemacytometer with phase microscope to make the count. In order to count the spleen cells, 0.1 ml of the suspension was diluted to 1 ml with Methyl Violet and used with a haemacytometer and light microscope and the stained nuclei of the cells counted.

$10^8$ spleen cells were then mixed with $5 \times 10^7$ myeloma cells, the mixture washed in serum-free DMEM high in glucose and centrifuged and all the liquid removed. The resultant cell pellet was placed in a 37° C. water bath and there was added over the period of one minute 1 ml of a 50% wt. vol. solution of polyethylene glycol 1500 (PEG) in saline Hepes; pH of approximately 7.5, and the mixture gently stirred for approximately one and one-half minutes. There was then slowly added 10 ml of serum-free tissue culture medium DMEM, followed by the addition of up to 50 ml of such culture medium, centrifugation and removal of all the supernatant, and resuspension of the cell pellet in 10 ml of DMEM containing 18% by weight fetal calf serum.

Ten microliters of the mixture was placed in each of 500 wells of standard multiwell tissue culture plates. Each well contained 1.0 ml of the standard HAT medium (hypoxanthine, aminopterin, and thymidine) and a feeder layer of Balb/c macrophages at a concentration of $5 \times 10^4$ macrophages/well.

The wells were kept undisturbed and cultured at 37° C. in 9% $CO_2$ air at approximately 100% humidity. The wells were then analyzed for growth utilizing the conventional inverted microscope procedure after about 5 to 10 days. In those wells in which growth was present in the inhibiting HAT medium, screening tests for the specific monoclonal antibody were made utilizing the conventional enzyme immunoassay screening method described below. Somewhere around 10 days to 14 days after fusion, sufficient antibody against the antigen had been developed in at least one well.

D. **Cloning**

From those wells which yielded antibody against the antigen, cells are removed and cloned using the standard dilution method.

The clones were assayed by the enzyme immunoassay method to determine antibody production. A positive clone may be recloned.

E.    Monoclonal Selection

The monoclonal antibodies from the clones are screened by the standard techniques for binding to Hepatitis B virus surface antigen; prepared as in the immunization, and for specificity in a test battery of Hepatitis virus antigens and known trace contaminant antigens.

F.    Antibody Production

At least one of two methods is usually available for production (and purification).

In Method A, six Balb/c mice are primed with pristane and injected intraperitoneally with $10^7$ cells of each of the monoclonal antibody specific against the antigen.  Then the ascites fluid is harvested after the mice have reached the proper stage; the mice are swollen with fluid but still alive.  The cells are then centrifuged at 1200 g for approximately 10 minutes, the cells discarded, and the antibody rich ascites fluid collected.

The fluid is titrated, as noted above, to establish presence and level of antibody and purified.

Purification can be accomplished using the protein A-Sepharose method, or the ammonium sulphate/DEAE method.

More particularly, in the protein A-Sepharose method, about 10 ml of the ascites fluid are filtered through glass wool and centrifuged at 30,000 g for 10 minutes. The ascites is then diluted with twice its own volume of cold phosphate buffer (0.1 M sodium phosphate, pH 8.2) and the diluted ascites is loaded on to a 2 ml column of protein A-Sepharose which had previously been equilibrated with phosphate buffer. The column is washed with 40 ml of phosphate buffer and the monoclonal antibody is eluted with citrate buffer (0.1 M sodium citrate, pH 3.5) into sufficient 1M tris buffer, pH 9.0, to raise the pH immediately to about 7.5 The eluate is dialysed in 2 x 1000 ml PBS at +4°C.

In the ammonium sulphate/DEAE method, about 10 ml of the ascites fluid are filtered through glass wool and centrifuged at 30,000 g for 10 minutes. The ascites is then stirred at +4°C and an equal volume of cold, saturated ammonium sulphate added slowly. The mixture is stirred for a further 30 minutes after addition is complete. The precipitate is harvested by centrifugation at 10,000 g for 10 minutes. The precipitate is dissolved in a minimum volume of cold phosphate/EDTA buffer (20 mM sodium phosphate, 10 mM EDTA, pH 7.5, + 0.02% sodium azide). The solution is dialyzed versus 2x1000 ml of the same buffer at +4°C. The dialyzed, redissolved precipitate is centrifuged at 30,000 g for 10 minutes and applied to a 10 ml column of DEAE-cellulose, previously equilibrated in phosphate/EDTA buffer. The monoclonal antibody is eluted with phosphate/EDTA buffer.

In Method B, cells of the monoclonal antibody-producing line specific to the antigen are grown in batch tissue culture. DMEM, to which has been added 10% FCS, is used to support growth in mid-log phase, to 1 litre volume. The culture is allowed to overgrow, to allow maximum antibody production. The culture is then centrifuged at 1200 g for approximately 10 minutes. The cell/cell debris is discarded and the antibody-rich supernatant collected.

The fluid may then be titrated, as noted above, to establish presence and level of antibody, and purified.

Purification can be accomplished by a combination of batch ion-exchange chromatography, ammonium sulphate precipitation and either column ion-exchange chromatograph or protein A-Sepharose chromatography.

More particularly, to one litre of culture supernatant is added one litre of 0.05M sodium acetate buffer, pH 4.5, and 40 ml of SP-Sephadex, previously equilibrated in 0.1M sodium acetate buffer, pH 5.0. The suspension is stirred at +4°C for one hour. The SP-Sephadex is allowed to settle and the supernatant is decanted. The SP-Sephadex is packed in a column, washed with 60 ml of 0.1M acetate buffer, pH 5.0, and eluted with 60 ml of the same buffer plus 1M sodium chloride. The eluate is stirred at +4°C, and an equal volume of saturated ammonium sulphate added slowly. The suspension is stirred for a further 30 minutes. The precipitate is then harvested by centrifugation at 10,000 g for 10 minutes. The precipitate is dissolved in a minimum volume of either cold phosphate/EDTA buffer (20mM sodium phosphate, 10mM EDTA, pH 7.5, + 0.02% sodium azide) for DEAE-cellulose chromatography, or phosphate buffer (0.1M sodium phosphate, pH 8.2 + 0.01% sodium azide) for protein A-Sepharose chromatography. The dissolved precipitate is dialysed versus 2 x 1000 ml of the dissolution buffer at +4°C, and the appropriate chromatography step carried out as previously described in Method A.

## G. Enzyme-Monoclonal Linkage

The monoclonal antibody specific against the antigen, prepared and screened as described above, is then bound to an appropriate enzyme such as a highly-purified alkaline phosphatase. This can be accomplished by any known method; e.g., the one-step glutaraldehyde method or benzoquinone conjugation.

In the glutaraldehyde method, monoclonal antibody (3 mg in about 1 ml of solution) is dialyzed with 10 mg of alkaline phosphatase (Sigma Type VII-T) against 2x1000 ml of phosphate buffered saline, pH 7.4 (PBS) at +4°C. After dialysis the volume is made up to 2.5 ml with PBS and 25 μl of a 20% glutaraldehyde in PBS solution added. The conjugation mixture was left at room temperature for 1.5 hours. After this time, glutaraldehyde is removed by gel filtration on a Pharmacia PH-10 (Sephadex G-25M) column, previously equilibrated in PBS. The conjugate is eluted with 3.5 ml PBS and then dialyzed against 2x2000 ml of TRIS buffer (50 mM TRIS, 1 mM magnesium chloride, pH 8.0 plus 0.02% sodium azide) at +4°C. To the dialyzed conjugate is added 1/10th its own volume of 10% BSA in TRIS buffer. The conjugate is then sterile filtered through a 0.22 μm membrane filter into a sterile amber vial and stored at +4°C.

In the benzoquinone method, 24 mg alkaline phosphatase (Sigma Type VII-T) are dialysed against 2 x 500 ml of 0.25M sodium phosphate buffer, pH 6.0, at +4°C. Para-benzoquinone, 18 mg, is dissolved in warm AR ethanol, 0.6 ml, and added to the dialysed alkaline phosphatase. The benzoquinone/alkaline phosphatase mixture is left in the dark at room temperature for 1 hour. After this time, unreacted benzoquinone and reaction by-products are removed and the buffer exchanged, by gel filtration on a Pharmacia PD-10 (Sephadex G-25M) column, previously equilibrated in 0.15M sodium chloride. The benzoquinone-activated alkaline phosphatase thus produced is sufficient for three 3 mg antibody conjugations.

3 mg monoclonal antibody are dialysed against 2 x 500 ml of 0.15M sodium chloride at +4°C. Dialysed antibody is added to 8 mg of benzoquinone-activated alkaline phosphatase, immediately followed by sufficient 1M sodium bicarbonate to give a final concentration of 0.1M. The

conjugation mixture is left in the dark at +4 C for 48 hours. After this time, sufficient 1M lysine is added to give a final concentration of 0.1M. After 2 hours in the dark at room temperature, the conjugate is dialysed against 2 x 1000 ml of phosphate-buffered saline + 0.02% sodium azide at +4 C. An equal volume of glycerol is added. The conjugate is sterile-filtered through a 0.22 μm membrane filter into a sterile amber vial, and stored at +4 C.

H.   Monoclonal Antibody Conjugate Testing

The enzyme immunoassay method is used for testing. This assay method comprises coating the wells of a standard polyvinyl chloride microtiter tray with the antigen, followed by addition of monoclonal antibody enzyme conjugate, and finally addition of the enzyme substrate, p-nitrophenyl phosphate. The monoclonal antibody, found to be specific for the antigen, can then be tested.

Four monoclonal antibodies have been tested; three of the class IgG1 and one of the class IgG2a have been found, and have been numbered as 80, 192, 329 and 341.

Antibody 80, of class IgG2a, can detect antigen at 5-10 ng/ml. It is the preferred antibody because of its affinity and specificity. 80 did not bind with the synthetic peptides corresponding to amino-acid sequences 124 to 137 and 139 to 147 of the Hepatitis B surface antigen. In like manner, 192 and 341 did not bind with sequence 139 to 147, although 192 did bind with sequence 124 to 137.

If deemed necessary, the particular epitopic site to which the antibody attaches to the antigen can also be determined. The same enzyme immunoassay method can also be used to determine whether diagnostic specimens such as urine, blood, stool, water or milk contain the antigen. In such cases, the antibody can first be bound to the plate.

CLAIMS

1.    An enzyme-labelled monoclonal antibody, in which the antibody has high binding affinity, is of immunological class IgG, is specific for the Hepatitis B surface antigen "a" determinants, and does not bind to the known biological "a" determinant corresponding to amino-acid sequence 139 to 147 of the Hepatitis B surface antigen.

2.    A labelled monoclonal antibody according to claim 1, wherein the enzyme is alkaline phosphatase, glucose oxidase, galactosidase or peroxidase.

3.    A labelled monoclonal antibody according to claim 2, wherein the enzyme is peroxidase.

4.    A labelled monoclonal antibody according to claim 1, wherein the antibody is of the immunological class IgG2a.

5.    A process for diagnosing for the presence of the surface antigen of Hepatitis B virus in a specimen, which comprises contacting the specimen with a labelled monoclonal antibody according to any preceding claim in an immunoassay procedure.

6.    A process according to claim 5, wherein the immunoassay procedure is an enzyme-linked immunoassay.